# EUROPEAN PATENT SPECIFICATION

(11) **EP 4 140 588 B1**
(45) Date of publication and mention of the grant of the patent: **12.08.2026**
(21) Application number: 21193715.6
(22) Date of filing: 30.08.2021
(51) Int. Cl.: B01L 3/02

(54) **PIPETTE TIP WITH TWO POSITIVE ANGLE STEPS**
PIPETTENSPITZE MIT ZWEI POSITIVEN WINKELSTUFEN
EXTRÉMITÉ DE PIPETTE AVEC DEUX ÉTAPES D'ANGLE POSITIF

(43) Date of publication of application: 01.03.2023
(73) Proprietor: Tecan Trading AG, 8708 Männedorf (CH)
(72) Inventor: Keller, Michael, Bauma (CH)
(74) Representative: Sykora & König Patentanwälte PartG mbB

(56) References cited:
- WO-A1-2014/179256
- WO-A1-2016/028998
- US-A- 5 223 225

## Description

### FIELD OF THE INVENTION

The invention relates to a pipette tip, for example for a laboratory automation device.

### BACKGROUND OF THE INVENTION

Laboratory automation devices are used for automating tasks of a laboratory assistant, which, for example, tests a patient for specific diseases. Usually, a sample of the patient's blood, urine, stool, etc. is taken and analyzed by means of a biochemical procedure. Such a procedure consists in various operations like adding substances, incubating, separating, etc. and a measurement process which quantitatively or qualitatively measures the amount or presence of a substance indicating the specific disease.

The samples are usually aspirated and dispensed with disposable pipette tips, which can be plugged onto nozzles of a handheld pipette or of a movable head of the laboratory automation devices. A handheld pipette may be actuated mechanically by the operator or it is actuated electronically. The geometric form of the pipette tips is crucial for an exact aspiration and dispensing process. Sometimes it is desirable that the amount of sample liquid aspirated and dispensed is controllable as exact as possible, in particular, when small volumes are aspirated and dispensed.

Several types of pipette tips have been designed subject to different needs with respect to the aspiration and dispensing process.

EP 3 272 851 B1 shows a pipette tip, which has a first cylindrical section with an orifice, a tapered section and a second cylindrical section with larger diameter than the first cylindrical section.

US 2019 0344 258 A1 shows a pipette tip, which has a conical section with an orifice and a cylindrical section.

EP 2 606 977 B1 shows several pipette tips composed of cylindrical and conical sections.

EP 3 492 926 B1 shows a pipette tip composed of conical sections with different opening angles.

WO 2016/028998 A1 and US 5 223 225 A show pipette tips with conical sections having increasing opening angle starting from the tip.

WO 2014/179256 A1 shows a pipette tip with conical and cylindrical sections.

### DESCRIPTION OF THE INVENTION

It is an objective of the invention to provide a pipette tip with which small amounts of liquid can be aspirated and dispensed exactly. A further objective of the invention is to increase the accuracy and precision of the amount of liquid being aspirated or dispensed with a laboratory automation device.

This objective is achieved by the subject-matter of claim 1. Further exemplary embodiments are evident from the dependent claims and the following description.

The invention relates to a pipette tip. In general, a pipette tip may be an elongated body with a cavity inside into which a liquid or other fluid can be aspirated or dispensed therefrom by changing a pressure in the cavity. Usually, pipette tips are composed of several sections with different inner diameters, which can be cylindrical and conical.

The pipette tip may be designed to be used with a laboratory automation device, for example may have an attachment section to be plugged onto a movable head of the laboratory automation device. However, the pipette tip also may be a part of a hand-held device.

According to the invention, the pipette tip comprises an end section, a first section, a second section and a third section aligned along a central axis of the pipette tip, wherein the end section comprises an orifice for aspirating and dispensing a fluid, in particular a liquid, emulsion or suspension. The pipette tip may be substantially rotationally symmetric with respect to the central axis. In particular, the end section, the first section, the second section and the third section, as well as the cylindrical section mentioned below may be rotationally symmetric.

According to the invention, a first kink is formed in an inner surface of the pipette tip between the first section and the second section, such that an angle between the inner surface of the first section and the central axis is smaller than an angle between the inner surface of the second section and the central axis. Additionally, a second kink is formed in the inner surface of the pipette tip between the second section and the third section, such that an angle between the inner surface of the second section and the central axis is smaller than an angle between the inner surface of the third section and the central axis.

Between the kinks, the inner surface of the pipette tip may be continuous and/or may have no or nearly no curvature. A kink also may be characterized by a region, where the curvature of the inner surface is rather high and/or where the radius of curvature is small. The curvature of the inner surface at the kinks is substantially higher (such as at least 10 times higher) as the curvature of the inner surface between the kinks.

Each of the first and second kink and also the kinks mentioned below may circumvent the central axis and/or may have a circular shape with circle center at the central axis.

The angle of a surface with respect to the central axis may be defined as the angle between a line inside the surface and the central axis, wherein the line is the cross-section of the surface with a plane going through the central axis.

In other words, the angles of the inner surfaces of the first, second and third section, which inner surfaces may be rotational symmetric with respect to the central axis, increase in this order. In such a way, two kinks are formed, which may be seen as positive kinks, i.e. they are protruding towards the inside of the pipette tip. When the fluid level passes one of these kinks during aspiration or dispensing, the border of the surface of the fluid stays or sticks at the kink due to capillary forces, even when the pressure inside the tip changes. There may be a range of volumes, which can be aspirated or dispensed, all of which have a fluid level at the respective kink. This effect can be used to better control the amount of fluid inside the pipette tip. In particular at the kinks, the amount of fluid aspirated and dispensed can be controlled with pressure changes rather accurately, since the border of the surface of the fluid does not move and is not subjected to uncontrollable movements caused by capillary forces.

A corresponding effect may take place at negative kinks, i.e. kinks that protrude away from the inside of the pipette tip. There, the liquid level may increase and decrease without a pressure change resulting in uncontrollable ranges of volume. Depending on the liquid and the tip material, a combination of a positive and a negative kink may be favorable.

The pipette tip as described herein solely has positive kinks in an end part comprising the first, second and third section. Thus, at lower volumes, the control of the aspirated and dispensed volume is improved.

According to the invention, an angle between the inner surface of the first section and the inner surface of the second section, i.e. the angle at the first kink, is larger than 1° and is smaller than 5°. Additionally, an angle between the inner surface of the second section and the inner surface of the third section, i.e. the angle at the second kink, is larger than 1° and is smaller than 5°. Experiments and tests have been shown that an angle at a kink should be larger than 1°. Otherwise, the above mentioned effect of staying or sticking of the fluid surface becomes too weak. Also, for angles of more than 5°, the effect usually becomes too strong to be beneficial.

It has to be noted that a real angle size can deviate by up to 10% of the angle size provided here, i.e. the angle of 1° mentioned above can be a real, measured angle from 0.9° to 1.1°. The same applies to the angles mentioned below.

According to an embodiment of the invention, the angle between the inner surface of the first section and the central axis is larger than 2° and/or smaller than 5°, in particular 3°. The angle between the inner surface of the second section and the central axis may be larger than 3° and/or smaller than 9°, in particular 5°. The angle between the inner surface of the third section and the central axis may be larger than 4° and/or smaller than 13°, in particular 6,5°.

Further tests showed that these absolute angles (compared to the relative angles between the sections) resulted in well controllable volumes within the first, second and third section of the pipette tip.

According to an embodiment of the invention, the position of the first kink between the first section and the second section is chosen, such that an inner volume of the pipette tip between the orifice and the first kink is between 0,3 µl and 0,6 µl, in particular 0,5 µl. The first pipetted volume may be fixed to 0,5 µl. As a further example, the first pipetted volume may be about 1,0 µl.

The position of the second kink between the second section and the third section is chosen, such that an inner volume of the pipette tip between the orifice and the second kink is between 0,8 µl and 1,2 µl, in particular 1,0 µl. The second pipetted volume may be fixed to 1,0 µl. The second pipetted volume be 2 µl or even 5 µl. Here also a range between 4,5 µl to 5,5 µl may be chosen.

As mentioned above, at the kinks, the volume of the fluid in pipette can be controlled very accurately. When positioning the kinks at positions, where the pipette has a specific inner volume, the aspiration and dispensing of an amount of the fluid having the specific inner volume can be controlled more accurately.

According to an embodiment of the invention, a length of the third section along the central axis is chosen, such that an inner volume of the pipette tip between the orifice and the length is at least a nominal volume of the pipette tip, such that at least 5 µl, or at least 10 µl. The third section may be used for aspirating much higher amount of fluid than the first and second section. The length of the third section also may be chosen such that an inner volume of the pipette tip between the orifice and the length is at least 5 times an inner volume of the pipette tip between the orifice and the second kink.

It also may be that a cylindrical section following the third section is present and that the nominal volume of the pipette is defined including the inner volume of the cylindrical section. In the case one defined volume is desired, the third section may be skipped and then the second section and/or a cylindrical section following the second section may be designed to allow bigger volumes.

As already mentioned, the inner surfaces of the pipette tip may have no or nearly no curvature between the kinks. Such surfaces may be defined by frustum of cones and cylinders.

According to an embodiment of the invention, the inner surface of the first section is conical, the inner surface of the second section is conical and/or the inner surface of the third section is conical. In this context, "conical" means frustoconical in a mathematical sense.

The end section of the pipette tip may have a conical inner surface and the inner surface between the end section and the first section of the pipette tip may have a further kink.

According to an embodiment of the invention, a further kink is formed in the inner surface of the pipette tip between the end section and the first section.

According to an embodiment of the invention, an angle between the inner surface of the end section and the inner surface of the first section is larger than 2° and/or is smaller than 5°.

According to an embodiment of the invention, the inner surface of the end section is cylindrical. The inner surface of the end section may have the same radius as the orifice, which may be defined by a border of the inner surface.

It has to be noted that an inner surface with a cylindrical shape may have an angle up to 0.5° with respect to the central axis due to reasons of manufacturing. For example, if the pipette tip is made by injection moulding, it may be necessary that the inner surface deviates a bit from pure cylindrical in the mathematical sense, such that a mould tool can be removed from the pipette tip after moulding.

According to an embodiment of the invention, a common outer surface of the first section, the second section and the third section and optionally the end section is conical. An angle of the outer surface with respect to the central axis may be the angle of the third section with respect to the central axis. Thus, a wall thickness of the pipette tip may increase in the end section, the first section and the second section.

According to an embodiment of the invention, the pipette tip comprises an attachment section for attaching the pipette tip to a nozzle of a laboratory automation device for changing a pressure inside the pipette tip. The nozzle may be a part of a moving head of the laboratory automation device. The attachment section is aligned along the central axis of the pipette tip following the third section and optionally a cylindrical section (see below). The attachment section may have a larger inner diameter than the third section and/or the cylindrical section. An angle between the inner surface of the attachment section and the central axis may be smaller than an angle between the inner surface of the third section and the central axis.

The attachment section also may have longitudinal ribs at an outside of the pipette tip for reinforcing the wall of the pipette tip there.

According to an embodiment of the invention, the attachment section is formed, such that a further pipette tip of the same shape is partially insertable into the attachment section to at least the third section, for stacking the further pipette tip and the pipette tip.

According to an embodiment of the invention, the pipette tip comprises a cylindrical section between the attachment section and the third section aligned along a central axis of the pipette tip and following the third section. The cylindrical section may have a length, which is larger than a length of the pipette tip between the orifice and the beginning of the cylindrical section. It even may be that the length of the cylindrical section is at least two times or at least five times larger than the of the pipette tip between the orifice and the beginning of the cylindrical section.

When the first and second kink (and also the further kink between the end section and the first section) are considered as positive kinks, where the angle increases, a negative kink is formed between the third section and the cylindrical section, where the angle decreases with increasing distance from the orifice.

According to an embodiment of the invention, the pipette tip comprises a filter section, which may be a part of the attachment section. The filter section is aligned along a central axis of the pipette tip and may follow the third section and/or the cylindrical section. The inner surface of the filter section may comprise ribs extending radially around the central axis. The filter section may have a larger inner diameter than the third section and/or the cylindrical section.

It has to be noted that the pipette tip may comprise more than three sections and/or more than two optimized volumes. Also solely one optimized volume is possible.

These and other aspects of the invention will be apparent from and elucidated with reference to the embodiments described hereinafter.

### BRIEF DESCRIPTION OF THE DRAWINGS

Below, embodiments of the present invention are described in more detail with reference to the attached drawings.
Fig. 1 shows a cross sectional view of a pipette tip according to an embodiment of the invention.
Fig. 2 shows an enlarged view of an end of the pipette tip of Fig. 1.
Fig. 3 shows a cross sectional view of two pipette tips according to Fig. 1 stacked into each other.
Fig. 4 shows a cross sectional view of a pipette tip according to a further embodiment of the invention.

The reference symbols used in the drawings, and their meanings, are listed in summary form in the list of reference symbols. In principle, identical parts are provided with the same reference symbols in the figures.

### DETAILED DESCRIPTION OF EXEMPLARY EMBODIMENTS

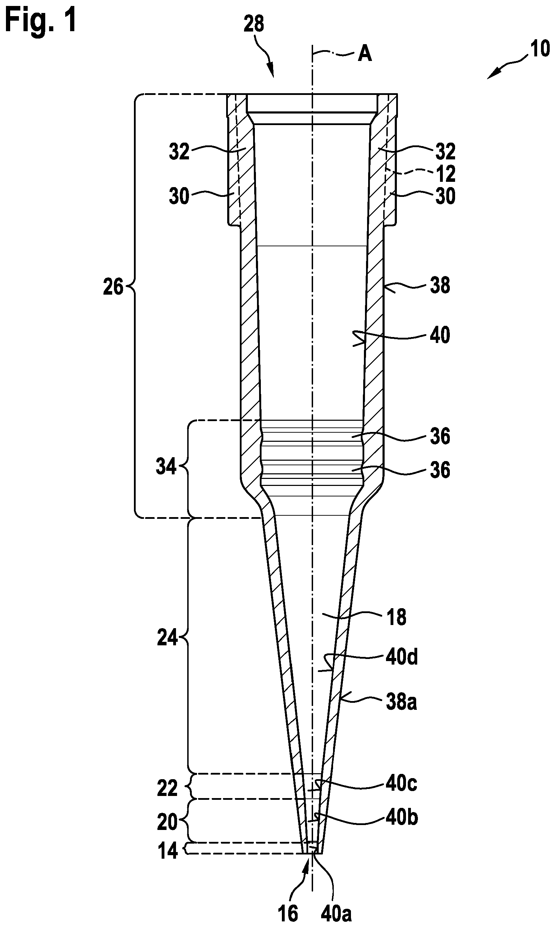

Fig. 1 shows a pipette tip 10, which can be used with a manual pipette or laboratory automation device. The pipette tip 10 comprises a substantially rotational symmetric body 12 with respect to a central axis A.

The pipette tip 10 can be divided into several sections of different geometric forms.

An end section 14 comprises an orifice 16 for aspirating a fluid into an inner cavity 18 of the pipette tip 10. The orifice 16, which may be circular, may have a diameter between 0.3 mm and 1.0 mm and/or the outer end of the pipette tip 10 may have a diameter of about 0.8 mm. The end section 14 may have a length of about 0.5 mm.

Along the axis A, a first section 20, second section 22 and third section 24 follows the end section 14 in this order. All of these sections as well as the end section are rotational symmetric with respect to the central axis A and/or have a circular cross-section orthogonal to the central axis A. Also, slightly oval cross-sections are possible. The first section 20 has a length of about 2.0 mm, the second section 22 of about 1.2 mm and the third section of about 12.4 mm. Here and in the following, the lengths are dependent on desired volumes for the respective sections in dependence of the diameters of the respective sections.

The end section 14, the first section 20, the second section 22 and the third section 24 are rotational symmetric with respect to the axis A. Again, slightly oval cross-sections are possible.

The pipette tip 10 furthermore comprises an attachment section 26 with an opening 28 opposite to the orifice 16. The attachment section 26 is designed for being connected to a nozzle of the a laboratory automation device or handheld pipette , which nozzle may be plugged into the opening 28. At an outer end, the attachment section 26 comprises ribs 30, which extend substantially parallel to the central axis A and which are provided on an outside of the pipette tip 10 and its body 12. In Fig. 1, a cross-section through a pair of opposite ribs 30 is shown. The thickness of a wall 32 of the attachment section 26 besides the ribs 30 is like the thickness of the wall 32 in a region, where no ribs 30 are present any more, i.e. towards the orifice 16.

The attachment section 26 has a length of about 19 mm. Except the ribs, the attachment section 26 is rotational symmetric with respect to the axis A.

The attachment section 26 is connected to the third section 24. At an end connected to the third section 24, the attachment section 26 comprises a filter section 34. The filter section 34 is designed for receiving a cylindrical filter piece and/or has inner ribs 36, which circumvent the central axis A on an inside of the pipette tip 10. The filter section 34 has a length of about 4 mm. The filter section 34 is rotational symmetric with respect to the axis A.

The pipette tip 10 and its body 12 may be made of plastics material. The plastic material may be conductive e. g. by graphite additives or may be non-conductive e. g. polypropylene. The pipette tip 10 may be manufactured by injection moulding, wherein an outer surface 38 is formed by an outer mould tool and an inner surface 40 is formed by an inner mould tool.

The inner surface 40a of the end section 14 is cylindrical and has the same diameter like the orifice 16. The inner surface 40b of the first section 20, the inner surface 40c of the second section 22 and the inner surface 40d of the third section 24 is conical. The terms "cylindrical" and "conical" relate to the shell surfaces of the respective body. There also may be pipette tips without a cylindrical end section. Such tips may have a very short end section, which may be the lower end of the first section, which provides the orifice.

It may be that the end of the inner surface 40d of the third section, which goes over into the inner surface of the attachment section 26, opens up shaped like a trumpet, i.e. with continuously increasing surface angle with respect to the central axis A.

An inner diameter of the first section 20, second section 22 and third section 24 is continuously increasing with increasing distance from the orifice 16. The angle with respect to the central axis A may be constant within a section. The angle increases from section to section with growing distance to the end section.

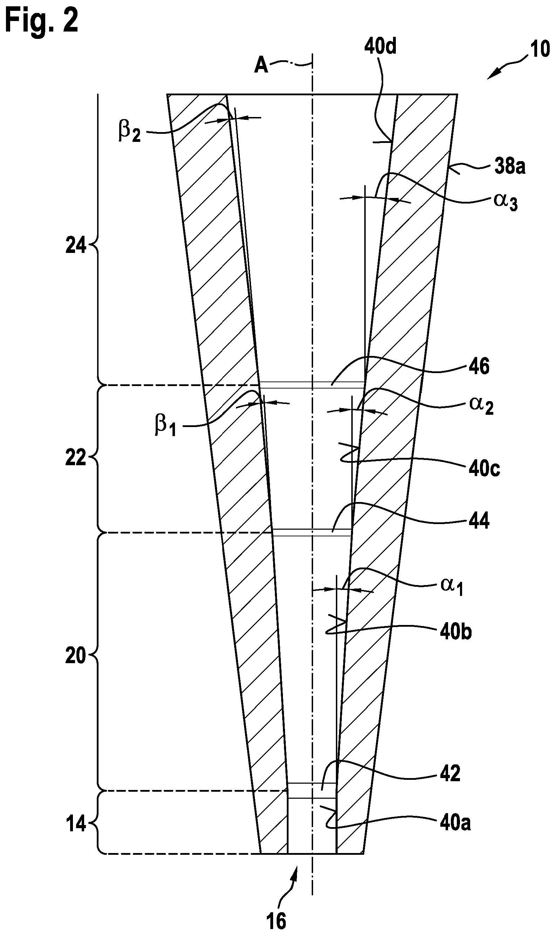

Fig. 2 shows an enlarged view of the sections 14, 20, 22, 24 of the pipette tip 10. It can be seen that a kink 42 is formed between the inner surface 40a of the end section 14 and the inner surface 40b of the first section 20, that a kink 44 is formed between the inner surface 40b of the first section 20 and the inner surface 40c of the second section 22 and that a kink 46 is formed between the inner surface 40c of the second section 22 and the inner surface 40d of the third section 24.

As described above, the accuracy and precision of the amount of fluid to be aspirated and dispensed, when the pipette tip is filled up to the respective kink 44, 46, can be increased. The positions of the kinks 44, 46 can be chosen, such that the accuracy at specific volumes is increased.

In the embodiment shown in Fig. 1 and 2, the position of the kink 44 is chosen, such that an inner volume of the pipette tip 10 between the orifice 16 and the kink 44 is about 0.5 µl. Also, the position of the second kink 46 is chosen, such that an inner volume of the pipette tip 10 between the orifice 16 and the second kink 46 is about 1.0 µl.

The third section 24 may be substantially longer than the first section 20 and the second section 22 and/or may have a substantially larger inner volume. The length of the third section 24 along the central axis A is chosen, such that an inner volume of the pipette tip 10 between the orifice 16 and the length is at least a nominal volume of the pipette tip 10, such that as more than 5 µl, for example 10 µl.

The kinks 42, 44, 46 are formed by a sudden step in the angle between the inner surface 40 of the pipette tip and the axis A. Each of the angles α₁, α₂, α₃, which are present between the central axis A and the surfaces 40b, 40c, 40d, is higher than the previous one, i.e. the inner surface 40 of the pipette tip 10 opens up at every kink 42, 44, 46. The kink 42 solely may be present in the case of a cylindrical end section 14.

The angle α₁ between the inner surface 40b of the first section 20 and the central axis A is smaller than the angle α₂ between the inner surface 40c of the second section 22 and the central axis A. The angle α₂ between the inner surface 40c of the second section 22 and the central axis A is smaller than the angle α₃ between the inner surface 40d of the third section 24 and the central axis A.

As described above, the following ranges of angles have been shown to achieve the beneficial effects of increasing the controllability of aspirating and dispensing and the accuracy at the volumes defined by the kinks 44, 46.

The angle α₁ between the inner surface 40b of the first section 20 and the central axis A may be larger than 2° and/or smaller than 5°, such as 3°. The angle α₂ between the inner surface 40c of the second section 22 and the central axis A may be larger than 3° and/or smaller than 9°, such as 5°. The angle α₃ between the inner surface 40d of the third section 24 and the central axis A may be larger than 4° and/or smaller than 13°, such as 6.5°.

The angle β₁ between the inner surface 40b of the first section 20 and the inner surface 40c of the second section 22 is the difference of the angles α₂ and α₁. The angle β₁ is larger than 1° and is smaller than 5°. The angle β₂ between the inner surface 40c of the second section 22 and the inner surface 40d of the third section 24 is the difference of the angles α₃ and α₂. The angle β₂ is larger than 1° and is smaller than 5°.

It is not necessary that the outer surface 38 of the pipette tip 10 also has kinks. In the present embodiment, a common outer surface 38a of the end section 14, the first section 20, the second section 22 and the third section 24 is conical.

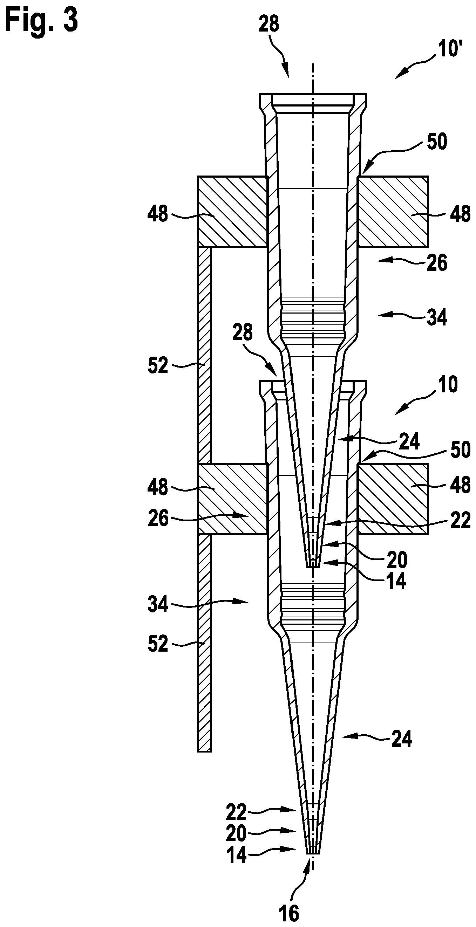

Fig. 3 shows the pipette tip 10 of Fig. 1 and 2 and a further pipette tip 10' of the same design. It is shown that the attachment section 26 is formed, such that the further pipette tip 10' is partially insertable into the attachment section 26 to at least the third section, for stacking the further pipette tip 10' and the pipette tip 10. The pipette tips 10, 10' hang in a tray 48 with holes 50. The holes 50 have a smaller diameter than the outside diameter of the ribs 30 and a larger diameter than the outside diameter of the coupling section without ribs 30. The trays 48 are kept at a distance by spacers 52 so that the space for stacking is optimally used.

The third section 24, which has the largest outer diameter of the sections 14, 20, 22, 24, has an outer diameter smaller than the inner diameter of the attachment section 26 at the opening 28. Furthermore, the length of the attachment section 26 (without the optional filter section 34) is at least 80% of the overall length of the sections 14, 20, 22, 24.

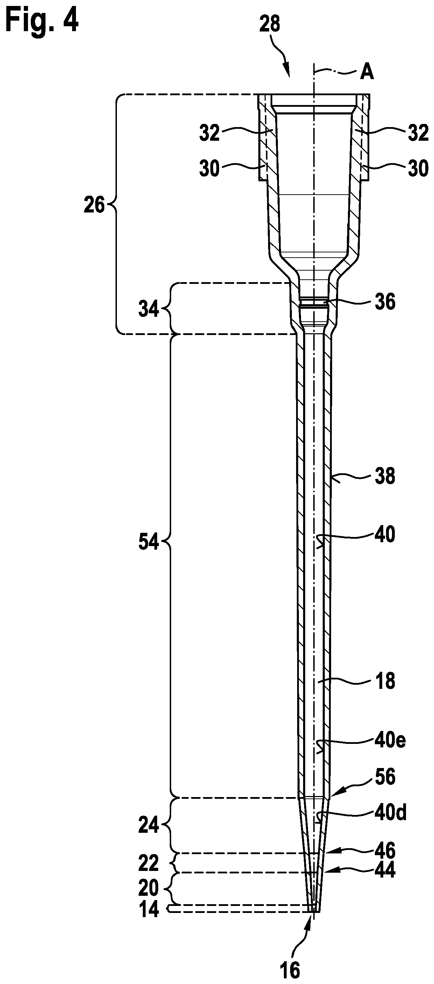

Fig. 4 shows a pipette tip 10, which additionally comprises a cylindrical section 54 between the third section 24 and the attachment section 26. With the cylindrical section 54, the nominal volume of the pipette 10 can be substantially increased. The cylindrical section 54 is longer than the pipette tip 10 between the orifice 16 and the cylindrical section 54, i.e. longer than the sections 14, 20, 22, 24.

The other parts of the pipette tip 10 of Fig. 4 can be designed as the corresponding parts of pipette tip 10 of Fig. 1 to 3. In particular, the pipette tip 10 of Fig. 4 also comprises the kinks 44, 46, which increase the accuracy in aspirating and dispensing as described above.

The pipette tip 10 of Fig. 4 comprises a kink 56 between the inner surface 40d of the third section 24 and the inner surface 40e of the cylindrical section 54, which however protrudes away from the central axis A contrary to the kinks 42, 44, 46.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### LIST OF REFERENCE SYMBOLS

- A: central axis
- 10: pipette tip
- 12: body
- 14: end section
- 16: orifice
- 18: inner cavity
- 20: first section
- 22: second section
- 24: third section
- 26: attachment section
- 28: opening
- 30: outer ribs
- 32: wall
- 34: filter section
- 36: inner ribs
- 38: outer surface
- 38a: outer surface
- 40: inner surface
- 40a: inner surface of 14
- 40b: inner surface of 20
- 40c: inner surface of 22
- 40d: inner surface of 24
- 42: kink between 40a and 40b
- 44: kink between 40b and 40c
- 46: kink between 40c and 40d
- α₁: angle between A and 40b
- α₂: angle between A and 40c
- α₃: angle between A and 40d
- β₁: angle between 40b and 40c
- β₂: angle between 40c and 40d
- 48: tray
- 50: hole
- 52: spacer
- 54: cylindrical section
- 40e: inner surface of 54
- 56: kink

## Claims

1. A pipette tip (10) comprising:
an end section (14), a first section (20), a second section (22) and a third section (24) aligned along a central axis (A) of the pipette tip (10);
wherein the end section (14) comprises an orifice (16) for aspirating and dispensing a fluid;
wherein a first kink (44) is formed in an inner surface (40) of the pipette tip (10) between the first section (20) and the second section (22), such that an angle (α₁) between the inner surface (40b) of the first section (20) and the central axis (A) is smaller than an angle (α₂) between the inner surface (40c) of the second section (22) and the central axis (A);
wherein a second kink (46) is formed in the inner surface (40) of the pipette tip (10) between the second section (22) and the third section (24), such that the angle (α₂) between the inner surface (40c) of the second section (22) and the central axis (A) is smaller than an angle (α₃) between the inner surface (40d) of the third section (24) and the central axis (A);
**characterised in that** an angle (β₁) between the inner surface (40b) of the first section (20) and the inner surface (40c) of the second section (22) is larger than 1° and is smaller than 5°;
wherein an angle (β₂) between the inner surface (40c) of the second section (22) and the inner surface (40d) of the third section (24) is larger than 1° and is smaller than 5°.

2. The pipette tip (10) of claim 1,
wherein the angle (α₁) between the inner surface (40b) of the first section (20) and the central axis (A) is larger than 2° and/or smaller than 5°;
wherein the angle (α₂) between the inner surface (40c) of the second section (22) and the central axis (A) is larger than 3° and/or smaller than 9°;
wherein the angle (α₃) between the inner surface (40d) of the third section (24) and the central axis (A) is larger than 4° and/or smaller than 13°.

3. The pipette tip (10) of one of the previous claims,
wherein the position of the first kink (44) is chosen, such that an inner volume of the pipette tip (10) between the orifice (16) and the first kink (44) is between 0.3 µl and 0.6 µl; and/or
wherein the position of the second kink (46) is chosen, such that an inner volume of the pipette tip (10) between the orifice (16) and the second kink (46) is between 0.8 µl and 1.2 µl.

4. The pipette tip (10) of one of the previous claims,
wherein a length of the third section (24) along the central axis (A) is chosen, such that an inner volume of the pipette tip (10) between the orifice (16) and the length is at least a nominal volume of the pipette tip (10), such that at least 5 µl.

5. The pipette tip (10) of one of the previous claims,
wherein the inner surface (40b) of the first section (20) is conical;
wherein the inner surface (40c) of the second section (22) is conical;
wherein the inner surface (40d) of the third section (24) is conical.

6. The pipette tip (10) of one of the previous claims,
wherein a further kink (42) is formed in the inner surface (40) of the pipette tip (10) between the end section (14) and the first section (20).

7. The pipette tip (10) of one of the previous claims,
wherein an angle (α₁) between the inner surface (40a) of the end section (14) and the inner surface (40b) of the first section (20) is larger than 2° and/or is smaller than 5°.

8. The pipette tip (10) of one of the previous claims,
wherein the inner surface (40a) of the end section (14) is cylindrical.

9. The pipette tip (10) of one of the previous claims,
wherein a common outer surface (38a) of the first section (20), the second section (22) and the third section (24) is conical.

10. The pipette tip (10) of one of the previous claims,
wherein the pipette tip (10) comprises an attachment section (26) for attaching the pipette tip (10) to a nozzle of a laboratory automation device for changing a pressure inside the pipette tip (10);
wherein the attachment section (26) is aligned along the central axis (A) of the pipette tip (10) following the third section (24).

11. The pipette tip (10) of claim 8 or 9,
wherein the attachment section (26) is formed, such that a further pipette tip (10') is partially insertable into the attachment section (26) to at least the third section, for stacking the further pipette tip (10') and the pipette tip (10).

12. The pipette tip (10) of one of the previous claims,
wherein the pipette tip (10) comprises a cylindrical section (54) aligned along the central axis (A) of the pipette tip (10) following the third section (24);
wherein the cylindrical section (54) is longer than the pipette tip (10) between the orifice (16) and the cylindrical section (54).

13. The pipette tip (10) of one of the previous claims,
wherein the pipette tip (10) comprises a filter section (34) aligned along the central axis (A) of the pipette tip (10), the filter section (34) following the third section (24);
wherein the inner surface (40) of the filter section (34) comprises ribs (36) extending radially around the central axis (A).

## Patentansprüche

1. Pipettenspitze (10), umfassend:
eine Endsektion (14), eine erste Sektion (20), eine zweite Sektion (22) und eine dritte Sektion (24), die entlang einer Mittelachse (A) der Pipettenspitze (10) ausgerichtet sind;
wobei die Endsektion (14) eine Öffnung (16) zum Ansaugen und Abgeben eines Fluids umfasst;
wobei an einer Innenfläche (40) der Pipettenspitze (10) zwischen der ersten Sektion (20) und der zweiten Sektion (22) ein erster Knick (44) gebildet ist, dergestalt, dass ein Winkel (α₁) zwischen der Innenfläche (40b) der ersten Sektion (20) und der Mittelachse (A) kleiner ist als ein Winkel (α₂) zwischen der Innenfläche (40c) der zweiten Sektion (22) und der Mittelachse (A);
wobei an der Innenfläche (40) der Pipettenspitze (10) zwischen der zweiten Sektion (22) und der dritten Sektion (24) ein zweiter Knick (46) gebildet ist, dergestalt, dass der Winkel (α2) zwischen der Innenfläche (40c) der zweiten Sektion (22) und der Mittelachse (A) kleiner ist als ein Winkel (α₃) zwischen der Innenfläche (40d) der dritten Sektion (24) und der Mittelachse (A);
**dadurch gekennzeichnet, dass**
ein Winkel (β₁) zwischen der Innenfläche (40b) der ersten Sektion (20) und der Innenfläche (40c) der zweiten Sektion (22) größer als 1° ist und kleiner als 5° ist;
wobei ein Winkel (β₂) zwischen der Innenfläche (40c) der zweiten Sektion (22) und der Innenfläche (40d) der dritten Sektion (24) größer als 1° ist und kleiner als 5° ist.

2. Pipettenspitze (10) nach Anspruch 1,
wobei der Winkel (α₁) zwischen der Innenfläche (40b) der ersten Sektion (20) und der Mittelachse (A) größer als 2° und/oder kleiner als 5° ist;
wobei der Winkel (α₂) zwischen der Innenfläche (40c) der zweiten Sektion (22) und der Mittelachse (A) größer als 3° und/oder kleiner als 9° ist;
wobei der Winkel (α₃) zwischen der Innenfläche (40d) der dritten Sektion (24) und der Mittelachse (A) größer als 4° und/oder kleiner als 13° ist.

3. Pipettenspitze (10) nach einem der vorangehenden Ansprüche,
wobei die Position des ersten Knicks (44) so gewählt ist, dass ein Innenvolumen der Pipettenspitze (10) zwischen der Öffnung (16) und dem ersten Knick (44) zwischen 0,3 µl und 0,6 µl beträgt; und/oder
wobei die Position des zweiten Knicks (46) so gewählt ist, dass ein Innenvolumen der Pipettenspitze (10) zwischen der Öffnung (16) und dem zweiten Knick (46) zwischen 0,8 µl und 1,2 µl beträgt.

4. Pipettenspitze (10) nach einem der vorangehenden Ansprüche,
wobei eine Länge der dritten Sektion (24) entlang der Mittelachse (A) so gewählt ist, dass ein Innenvolumen der Pipettenspitze (10) zwischen der Öffnung (16) und der Länge mindestens ein Nennvolumen der Pipettenspitze (10), wie mindestens 5 µl, beträgt.

5. Pipettenspitze (10) nach einem der vorangehenden Ansprüche,
wobei die Innenfläche (40b) der ersten Sektion (20) konisch ist;
wobei die Innenfläche (40c) der zweiten Sektion (22) konisch ist;
wobei die Innenfläche (40d) der dritten Sektion (24) konisch ist.

6. Pipettenspitze (10) nach einem der vorangehenden Ansprüche,
wobei an der Innenfläche (40) der Pipettenspitze (10) zwischen der Endsektion (14) und der ersten Sektion (20) ein weiterer Knick (42) gebildet ist.

7. Pipettenspitze (10) nach einem der vorangehenden Ansprüche,
wobei ein Winkel (α₁) zwischen der Innenfläche (40a) der Endsektion (14) und der Innenfläche (40b) der ersten Sektion (20) größer als 2° ist und/oder kleiner als 5° ist.

8. Pipettenspitze (10) nach einem der vorangehenden Ansprüche,
wobei die Innenfläche (40a) der Endsektion (14) zylindrisch ist.

9. Pipettenspitze (10) nach einem der vorangehenden Ansprüche,
wobei eine gemeinsame Außenfläche (38a) der ersten Sektion (20), der zweiten Sektion (22) und der dritten Sektion (24) konisch ist.

10. Pipettenspitze (10) nach einem der vorangehenden Ansprüche,
wobei die Pipettenspitze (10) eine Befestigungssektion (26) zum Befestigen der Pipettenspitze (10) an einer Düse einer Laborautomatisierungsvorrichtung zum Ändern eines Drucks im Inneren der Pipettenspitze (10) umfasst;
wobei die Befestigungssektion (26) entlang der Mittelachse (A) der Pipettenspitze (10) im Anschluss an die dritte Sektion (24) ausgerichtet ist.

11. Pipettenspitze (10) nach Anspruch 8 oder 9,
wobei die Befestigungssektion (26) so gebildet ist, dass eine weitere Pipettenspitze (10') teilweise in die Befestigungssektion (26) bis mindestens zu der dritten Sektion einsetzbar ist, um die weitere Pipettenspitze (10') und die Pipettenspitze (10) zu stapeln.

12. Pipettenspitze (10) nach einem der vorangehenden Ansprüche,
wobei die Pipettenspitze (10) eine zylindrische Sektion (54) umfasst, die entlang der Mittelachse (A) der Pipettenspitze (10) im Anschluss an die dritte Sektion (24) ausgerichtet ist;
wobei die zylindrische Sektion (54) länger als die Pipettenspitze (10) zwischen der Öffnung (16) und der zylindrischen Sektion (54) ist.

13. Pipettenspitze (10) nach einem der vorangehenden Ansprüche,
wobei die Pipettenspitze (10) eine Filtersektion (34) umfasst, die entlang der Mittelachse (A) der Pipettenspitze (10) ausgerichtet ist, wobei sich die Filtersektion (34) an die dritte Sektion (24) anschließt;
wobei die Innenfläche (40) der Filtersektion (34) Rippen (36) umfasst, die sich radial um die Mittelachse (A) herum erstrecken.

## Revendications

1. Embout de pipette (10) comprenant :
une section d'extrémité (14), une première section (20), une deuxième section (22), et une troisième section (24) alignées le long d'un axe central (A) de l'embout de pipette (10) ;
dans lequel la section d'extrémité (14) comprend un orifice (16) pour aspirer et distribuer un fluide ;
dans lequel un premier pli (44) est formé dans une surface intérieure (40) de l'embout de pipette (10) entre la première section (20) et la deuxième section (22), de telle sorte qu'un angle (α₁) entre la surface intérieure (40b) de la première section (20) et l'axe central (A) est inférieur à un angle (α₂) entre la surface intérieure (40c) de la deuxième section (22) et l'axe central (A) ;
dans lequel un second pli (46) est formé dans la surface intérieure (40) de l'embout de pipette (10) entre la deuxième section (22) et la troisième section (24), de telle sorte que l'angle (α₂) entre la surface intérieure (40c) de la deuxième section (22) et l'axe central (A) est inférieur à un angle (α₃) entre la surface intérieure (40d) de la troisième section (24) et l'axe central (A) ;
**caractérisé en ce que**
un angle (β₁) entre la surface intérieure (40b) de la première section (20) et la surface intérieure (40c) de la deuxième section (22) est supérieur à 1° et est inférieur à 5° ;
dans lequel un angle (β₂) entre la surface intérieure (40c) de la deuxième section (22) et la surface intérieure (40d) de la troisième section (24) est supérieur à 1° et est inférieur à 5°.

2. Embout de pipette (10) selon la revendication 1,
dans lequel l'angle (α₁) entre la surface intérieure (40b) de la première section (20) et l'axe central (A) est supérieur à 2° et/ou inférieur à 5° ;
dans lequel l'angle (α₂) entre la surface intérieure (40c) de la deuxième section (22) et l'axe central (A) est supérieur à 3° et/ou inférieur à 9° ;
dans lequel l'angle (α₃) entre la surface intérieure (40d) de la troisième section (24) et l'axe central (A) est supérieur à 4° et/ou inférieur à 13°.

3. Embout de pipette (10) selon l'une des revendications précédentes,
dans lequel la position du premier pli (44) est choisie de telle sorte qu'un volume intérieur de l'embout de pipette (10) entre l'orifice (16) et le premier pli (44) est compris entre 0,3 µl et 0,6 µl ; et/ou
dans lequel la position du second pli (46) est choisie de telle sorte qu'un volume intérieur de l'embout de pipette (10) entre l'orifice (16) et le second pli (46) est compris entre 0,8 µl et 1,2 µl.

4. Embout de pipette (10) selon l'une des revendications précédentes,
dans lequel une longueur de la troisième section (24) le long de l'axe central (A) est choisie de telle sorte qu'un volume intérieur de l'embout de pipette (10) entre l'orifice (16) et la longueur est au moins égal à un volume nominal de l'embout de pipette (10), tel qu'au moins 5 µl.

5. Embout de pipette (10) selon l'une des revendications précédentes,
dans lequel la surface intérieure (40b) de la première section (20) est conique ;
dans lequel la surface intérieure (40c) de la deuxième section (22) est conique ;
dans lequel la surface intérieure (40d) de la troisième section (24) est conique.

6. Embout de pipette (10) selon l'une des revendications précédentes,
dans lequel un pli supplémentaire (42) est formé dans la surface intérieure (40) de l'embout de pipette (10) entre la section d'extrémité (14) et la première section (20).

7. Embout de pipette (10) selon l'une des revendications précédentes,
dans lequel un angle (α₁) entre la surface intérieure (40a) de la section d'extrémité (14) et la surface intérieure (40b) de la première section (20) est supérieur à 2° et/ou est inférieur à 5°.

8. Embout de pipette (10) selon l'une des revendications précédentes,
dans lequel la surface intérieure (40a) de la section d'extrémité (14) est cylindrique.

9. Embout de pipette (10) selon l'une des revendications précédentes,
dans lequel une surface extérieure commune (38a) de la première section (20), de la deuxième section (22) et de la troisième section (24) est conique.

10. Embout de pipette (10) selon l'une des revendications précédentes,
dans lequel l'embout de pipette (10) comprend une section de fixation (26) pour fixer l'embout de pipette (10) à une buse d'un dispositif d'automatisation de laboratoire pour faire varier une pression à l'intérieur de l'embout de pipette (10) ;
dans lequel la section de fixation (26) est alignée le long de l'axe central (A) de l'embout de pipette (10) suivant la troisième section (24).

11. Embout de pipette (10) selon la revendication 8 ou 9,
dans lequel la section de fixation (26) est formée de telle sorte qu'un embout de pipette supplémentaire (10') peut être partiellement inséré dans la section de fixation (26) jusqu'à au moins la troisième section, pour empiler l'embout de pipette supplémentaire (10') et l'embout de pipette (10).

12. Embout de pipette (10) selon l'une des revendications précédentes,
dans lequel l'embout de pipette (10) comprend une section cylindrique (54) alignée le long de l'axe central (A) de l'embout de pipette (10) suivant la troisième section (24) ;
dans lequel la section cylindrique (54) est plus longue que l'embout de pipette (10) entre l'orifice (16) et la section cylindrique (54).

13. Embout de pipette (10) selon l'une des revendications précédentes,
dans lequel l'embout de pipette (10) comprend une section de filtre (34) alignée le long de l'axe central (A) de l'embout de pipette (10), la section de filtre (34) suivant la troisième section (24) ;
dans lequel la surface intérieure (40) de la section de filtre (34) comprend des nervures (36) s'étendant radialement autour de l'axe central (A).
